(19) Europäisches Patentamt

European Patent Office

Office européen des brevets

(11) **EP 1 712 565 A1**

(12) **EUROPEAN PATENT APPLICATION**
published in accordance with Art. 158(3) EPC

(43) Date of publication:
**18.10.2006 Bulletin 2006/42**

(51) Int Cl.:
*C07K 16/28* (2006.01)   *C12N 15/11* (2006.01)
*C12N 1/15* (2006.01)   *C12N 1/19* (2006.01)
*C12N 1/21* (2006.01)   *C12N 5/00* (2006.01)
*C12P 21/02* (2006.01)   *A61K 39/395* (2006.01)
*A61P 35/00* (2006.01)   *A61P 35/02* (2006.01)
*A61P 37/02* (2006.01)   *A61P 43/00* (2006.01)
*C12P 21/08* (2006.01)

(21) Application number: **04820311.1**

(22) Date of filing: **10.12.2004**

(86) International application number:
**PCT/JP2004/018501**

(87) International publication number:
**WO 2005/056603 (23.06.2005 Gazette 2005/25)**

(84) Designated Contracting States:
**AT BE BG CH CY CZ DE DK EE ES FI FR GB GR
HU IE IS IT LI LT LU MC NL PL PT RO SE SI SK TR**

(30) Priority: **12.12.2003 JP 2003415758**

(71) Applicant: **CHUGAI SEIYAKU KABUSHIKI KAISHA
Tokyo, 115-8543 (JP)**

(72) Inventors:
• **KIMURA, Naoki,
c/o CHUGAI SEIYAKU KABUSHIKI KAISHA
Ibaraki 300-4101 (JP)**
• **TSUCHIYA, M.,
c/o CHUGAI SEIYAKU KABUSHIKI KAISHA
Shizuoka 412-0038 (JP)**
• **NANAMI, M.,
c/o CHUGAI SEIYAKU KABUSHIKI KAISHA
Shizuoka 412-0038 (JP)**
• **TOMIMATSU, T.,
c/o CHUGAI SEIYAKU KABUSHIKI KAISHA
Tokyo 115-8543 (JP)**
• **KAWAI, Shigeto,
c/o CHUGAI SEIYAKU KABUSHIKI KAISH
Kanagawa 247-8530 (JP)**

(74) Representative: **Bassil, Nicholas Charles et al
Kilburn & Strode
20 Red Lion Street
London WC1R 4PJ (GB)**

(54) **CELL DEATH INDUCING AGENTS**

(57)   The present inventors constructed a DNA expression vector encoding 2D7sc(Fv)2 in which the heavy chain variable region sequence (VH) and the light chain variable region sequence (VL) of the 2D7 antibody is arranged in the order of VH-VL-VH-VL, and these sequences are linked by a 15-mer linker. The vector was introduced into CHO cells and a 2D7sc(Fv)2-producing expression cell line was established. When 2D7sc(Fv)2 was expressed in this cell line, purified, and cell death-inducing experiments performed, 2D7sc(Fv)2 was found to have a concentration-dependent cell death-inducing activity.

**Description**

Technical Field

The present invention relates to sc(Fv)2 of HLA-recognizing antibodies.

Background Art

The HLA class I antigen is formed by a heterodimer of a 45-KD α chain comprising three domains (α1, α2, α3), and a 12-KD β2 microglobulin. The main role of the HLA molecule is to present CD8⁺T cells with antigenic peptides formed from about eight to ten amino acids and produced inside cells. As such, it plays a very important role in the immune response and immune tolerance induced by this peptide presentation.

Cell growth-suppressing and cell death-inducing effects have been observed in lymphocytes upon HLA class IA antigen and antibody ligation, suggesting that HLA molecules may also be signal transduction molecules.

More specifically, for example, there are reports showing cell growth suppression of activated lymphocytes by the B9.12.1 antibody against the α1 domain of human HLA class IA, the W6/32 antibody against the α2 domain, and the TP25.99 and A1.4 antibodies against the α3 domain (non-patent literature 1, 2). Furthermore, two types of antibodies, MoAb90 and YTH862, against the human HLA class IA α1 domain have been reported to induce apoptosis in activated lymphocytes (non-patent literature 2, 3, 4). Apoptosis induced by these two antibodies has been shown to be a caspase-mediated reaction (non-patent literature 4), and therefore, HLA class IA antigens expressed in lymphocytes are also speculated to be involved in apoptosis signal transduction.

Furthermore, the 5H7 antibody against the α3 domain of human HLA class IA (non-patent literature 5), and the RE2 antibody against the α2 domain of mouse HLA class IA (non-patent literature 6) have been also reported to induce cell death in activated lymphocytes and the like. However, in contrast with the aforementioned apoptosis-inducing antibodies MoAb90 and YTH862, it has been shown that none of the cell deaths induced by these antibodies are caspase-mediated. Accordingly, cell deaths induced by 5H7 and RE2 are predicted to be of a type completely different from conventionally known apoptosis mechanisms.

As described above, there are numerous reports of the cell growth-suppressing actions and cell death-inducing actions of anti-HLA antibodies. However, the antibodies used herein are all in the molecular forms of IgG antibodies, F(ab')2, or Fab, and to date there have been no reports that cell death-inducing activity is enhanced by reducing the molecular weight of antibodies, as in F(ab')2 and Fab.

The 2D7 antibody is a mouse monoclonal antibody obtained by immunizing Balb/c mice with human myeloma cells (non-patent literature 7). The 2D7 antibody has been observed to bind highly specifically to the cell surface of various lymphoid tumor cells, however, antigens recognized by the 2D7 antibody have not been identified.

Prior art literature relating to the present invention of this application is shown below.

[Non-patent Document 1]     Fayen et al., Int. Immunol. 10: 1347-1358(1998)
[Non-patent Document 2]     Genestier et al., Blood 90: 3629-3639 (1997)
[Non-patent Document 3]     Genestier et al., Blood 90: 726-735 (1997)
[Non-patent Document 4]     Genestier et al., J. Biol. Chem. 273: 5060-5066 (1998)
[Non-patent Document 5]     Woodle et al., J. Immunol. 158: 2156-2164 (1997)
[Non-patent Document 6]     Matsuoka et al., J. Exp. Med. 181: 2007-2015 (1995)
[Non-patent Document 7]     Goto, et al. Blood 84: 1922 (1994)

Disclosure of the Invention

The present invention was achieved in view of the above circumstances. An objective of the present invention is to provide antibodies that recognize HLA class IA, and have a strong cell death-inducing activity as well as excellent stability in blood. More specifically, the objective is to provide antibodies comprising two heavy chain variable regions and two light chain variable regions, wherein the antibodies are single chain polypeptides comprising a binding activity against human leukocyte antigens (HLAs).

The present inventors conducted dedicated research to solve the above-mentioned objective. The 2D7 antibody is a mouse antibody obtained by a research group of the First Department of Internal Medicine, School of Medicine, University of Tokushima by immunizing mice with patient-derived leukemia cells. The present inventors have already filed a patent application (W02004/033499) for their discovery that the 2D7 antibody binds to the cell surface of various lymphoid tumor cells with a high specificity, and recognizes HLA-A. They also discovered that cell death-inducing activity increases when an anti-HLA antibody is converted to a low-molecular weight antibody, such as a diabody (WO2004/033499). Upon further dedicated research to increase antibody activity, the present inventors discovered that conversion of an antibody to a sc(Fv)2 makes it highly stable in blood while maintaining a superior activity. More specifically, a DNA expression vector encoding 2D7sc(Fv)2 was constructed so as to arrange the heavy chain variable region sequence (VH) and the light chain variable region sequence (VL) of the 2D7 antibody in a VH-VL-VH-VL fashion, linking the VH and VL regions by a 15-mer linker. Then the vector was introduced into CHO cells to establish a 2D7sc(Fv)2-producing (expressing) cell line. When cell death induction experiments were performed using purified 2D7sc(Fv)2 that had been expressed by the established cell line, it was revealed that 2D7sc(Fv)2 has a concentration-dependent outstanding cell death-inducing activity and is highly stable in blood. To further investigate the stability of 2D7sc(Fv)2 in blood, change of antibody concentration in mouse blood over time was analyzed. The results showed that the disappearance time of 2D7sc(Fv)2 in blood was significantly prolonged as compared to that of a 2D7 diabody. Therefore, 2D7sc(Fv)2 was found to be a low-molecular weight antibody that has a strong cell death-inducing activity and a cell growth-suppressing activity, which also exhibits excellent stability in blood.

More specifically, the present invention provides the following [1] to [28], and relates to antibodies comprising two heavy chain variable regions and two light chain variable regions, wherein the antibodies are single chain polypeptides comprising a binding activity against human leukocyte antigens (HLAs):

[1] an antibody comprising two heavy chain variable regions and two light chain variable regions, wherein the antibody is a single chain polypeptide having a binding activity against human leukocyte antigen (HLA);
[2] the antibody of [1], wherein the two heavy chain variable regions and two light chain variable regions are arranged in the order of heavy chain variable region, light chain variable region, heavy chain variable region, and light chain variable region, starting from the N terminus of the single chain polypeptide;
[3] the antibody of [1] or [2], wherein the two heavy chain variable regions and two light chain variable regions are linked by a linker;
[4] the antibody of [3], wherein the linker comprises 15 amino acids;
[5] the antibody of any one of [1] to [4], wherein HLA is HLA class I;
[6] the antibody of [5], wherein HLA class I is HLA-A;
[7] the antibody of any one of [1] to [6], wherein the antibody is sc(Fv)2;
[8] an sc(Fv)2 comprising heavy chain variable regions that comprise CDR1, 2, and 3 consisting of the amino acid sequences of SEQ ID NOs: 3, 4, and 5;
[9] an sc(Fv)2 comprising light chain variable regions that comprise CDR 1, 2, and 3 consisting of the amino acid sequences of SEQ ID NOs: 6, 7, and 8;
[10] an sc(Fv)2 comprising heavy chain variable regions that comprise CDR1, 2, and 3 consisting of the amino acid sequences of SEQ ID NOs: 3, 4, and 5, and light chain variable regions that comprise CDR 1, 2, and 3 consisting of the amino acid sequences of SEQ ID NOs: 6, 7, and 8;
[11] an sc(Fv)2 comprising heavy chain variable regions that comprise the amino acid sequence of SEQ ID NO: 10;
[12] an sc(Fv)2 comprising light chain variable regions that comprise the amino acid sequence of SEQ ID NO: 12;
[13] an sc(Fv)2 comprising heavy chain variable regions that comprise the amino acid sequence of SEQ ID NO: 10, and light chain variable regions that comprise the amino acid sequence of SEQ ID NO: 12;
[14] an sc(Fv)2 comprising the amino acid sequence of SEQ ID NO: 14;
[15] an sc(Fv)2 comprising the amino acid sequence of SEQ ID NO: 2;
[16] an sc(Fv)2 comprising an amino acid sequence with one or more amino acid substitutions, deletions, additions, and/or insertions in the amino acid sequence of any one of [8] to [15], wherein the sc(Fv)2 also has an activity equivalent to that of the antibody of any one of [8] to [15];
[17] a polynucleotide encoding the antibody of any one of [1] to [16];
[18] a polynucleotide that hybridizes with the polynucleotide of [17] under stringent conditions, and encodes an antibody having an activity equivalent to the antibody of any one of [1] to [16];
[19] a vector comprising the polynucleotide of [17] or [18];
[20] a host cell carrying the polynucleotide of [17] or [18], or the vector of [19];
[21] a method for producing the antibody of any one of [1] to [16], wherein the method comprises the steps of:

(a) preparing an HLA-recognizing antibody;
(b) producing a polynucleotide encoding the antibody of any one of [1] to [16] based on the sequence of the antibody prepared in (a);
(c) constructing a vector comprising the polynucleotide of (b);
(d) introducing the vector of (c) into host cells; and
(e) culturing the host cells of (d).

[22] a cell death-inducing agent comprising the antibody of any one of [1] to [16] as an active ingredient;
[23] the cell death-inducing agent of [22], wherein the agent has cell death inducing activity against B cells or T cells;

[24] the cell death-inducing agent of [23], wherein the B cells or T cells are activated B cells or activated T cells;
[25] a cell growth inhibitory agent comprising the antibody of any one of [1] to [16] as an active ingredient;
[26] an antitumor agent comprising the antibody of any one of [1] to [16] as an active ingredient;
[27] the antitumor agent of [26], wherein the tumor is a blood tumor; and
[28] a therapeutic agent for autoimmune diseases, wherein the agent comprises the antibody of any one of [1] to [16] as an active ingredient.

Brief Description of the Drawings

Fig. 1 shows structures of 2D7 low-molecular weight antibodies. Fig. 1 A shows a 2D7 diabody, and shows that the diabody (HL5) is formed as a result of a non-covalent bond-mediated dimerization of a heavy chain variable region (VE) and a light chain variable region (VL) linked by a 5-mer linker. Fig. 1B shows the sc(Fv)2 form. The sc(Fv)2 form takes the structure of B as a result of an internal folding of a single chain formed by linking two sets of VH-VL by a 15-mer linker.
Fig. 2 shows the nucleotide sequence and amino acid sequence of 2D7sc(Fv)2. The letters in bold italics indicate the signal sequence of the heavy chain variable region, the underlined sequences indicate the linker regions (15mer), and the bold letters at the C-terminus indicate the Flag tag region. Starting from the 5' end, the boxed nucleotide sequences indicate EcoRI, BamHI, and NotI restriction enzyme cleavage sites, respectively.
Fig.3 shows a comparison of chromatograms of 2D7 diabody and 2D7sc(Fv)2 upon purification by gel filtration chromatography. (1) shows the gel filtration elution chromatogram of 2D7sc(Fv)2, and (2) shows the gel filtration elution chromatogram of 2D7 diabody.
Fig. 4 compares *the in vitro* cell death-inducing activities of 2D7 diabody and 2D7sc(Fv)2.
Fig. 5 compares the cell growth-inhibitory activities of 2D7 diabody and 2D7sc(Fv)2.
Fig. 6 shows the change in radioactivity concentration of plasma TCA-precipitable fractions after a single intravenous administration of radiolabeled 2D7sc(Fv)2 and radiolabeled 2D7diabody (HL5) to mice.

Best Mode for Carrying Out the Invention

The present invention provides antibodies comprising two heavy chain variable regions and two light chain variable regions, wherein the antibodies are single chain polypeptides having a binding activity against human leukocyte antigens (HLAs). The antibodies of the present invention are useful because they have enhanced activity. Herein "activity" refers to a biological action that arises as a result of antigen-antibody binding. Specific examples include cell death-inducing actions, apoptosis-inducing actions, cell growth-suppressing actions, cell differentiation-suppressing actions, cell division-suppressing actions, cell growth-inducing actions, cell differentiation-inducing actions, cell division-inducing actions, and cell cycle-regulating actions. Cell death-inducing actions and cell growth-suppressing actions are preferred.
The cells that become the target of the above-mentioned actions, such as cell death-inducing actions and cell growth-suppressing actions, are not particularly limited, though blood cells and non-adherent cells are preferred. Specific examples of blood cells include lymphocytes (B cells, T cells), neutrophils, eosinophils, basophils, monocytes (preferably activated peripheral blood mononuclear cells (PBMC)), and myeloma cells, while lymphocytes (B cells, T cells), and myeloma cells are preferred, and T cells or B cells (particularly activated B cells or activated T cells) are most preferable. "Non-adherent cells" refer to cells that, when cultured, grow in a non-adherent state without adhering to the surface of culturing vessels made of glass, plastic or the like. On the other hand, "adherent cells" refer to cells that, when cultured, adhere to the surface of culturing vessels of glass, plastic or the like.
Generally, to exhibit cell death-inducing activity, a full length anti-HLA antibody has to crosslink with an anti-IgG antibody or such. However, the antibodies of the present invention can exhibit cell death-inducing activity without having to crosslink with an anti-IgG antibody.
Whether or not the antibodies of the present invention will induce cell death in non-adherent cells can be determined by observing induction of cell death in Jurkat cells or ARH77 cells. Whether or not the antibodies will induce cell death in adherent cells can be determined by observing induction of cell death in HeLa cells (W02004/033499).
In the present invention, administration of an antibody comprising two heavy chain variable regions and two light chain variable regions, wherein the antibody is a single chain polypeptide comprising binding activity against human leukocyte antigen (HLA) can treat or prevent diseases such as tumors including blood tumors (hematopoietic tumors) (specific examples include leukemia, myelodysplastic syndrome, malignant lymphoma, chronic myelogenic leukemia, plasmacytic disorders (myeloma, multiple myeloma, macroglobulinemia), and myeloproliferative diseases (polycythemia vera, essential thrombocythemia, idiopathic myelofibrosis)), and autoimmune diseases (specific examples include rheumatism, autoimmune hepatitis, autoimmune thyroiditis, autoimmune bullosis, autoimmune adrenocortical disease, autoimmune hemolytic anemia, autoimmune thrombycytopenic purpura, autoimmune atrophic gastritis, autoimmune neutropenia, autoimmune orchitis, autoimmune encephalomyelitis, autoimmune receptor disease, autoimmune infertility, Crohn's disease, systemic lupus erythematosus, multiple sclerosis, Basedow's disease, juvenile diabetes, Addison's disease, myasthenia gravis, lens-induced uveitis, psoriasis, and Behcet's disease). Furthermore, the excellent stability of the present invention's antibodies *in vivo* would be particularly efficacious when administering to a subject.

In the present invention, HLA refers to human leukocyte antigen. HLA molecules are categorized into class I and class II. Known examples of class I are HLA-A, B, C, E, F, G, H, J, and such; and known examples of class II are HLA-DR, DQ, DP, and such. The antigens recognized by the antibodies of this invention are not particularly limited, so long as they are HLA molecules, preferably molecules classified as class I, and more preferably HLA-A.

An antibody of the present invention is preferably an antibody comprising two heavy chain variable regions and two light chain variable regions which are aligned in the order of heavy chain variable region, light chain variable region, heavy chain variable region, and light chain variable region beginning from the N terminus of the single chain polypeptide. In a more preferable antibody, the two heavy chain variable regions and two light chain variable regions are linked by linkers. An example of such an antibody includes sc(Fv)2.

sc(Fv)2 is a single-chain polypeptide antibody, prepared by linking two sets of heavy chain variable region ([VH]) and two sets of light chain variable region ([VL]) with linkers and such (Hudson et al., J. Immunol. Methods 1999; 231: 177-189). sc(Fv)2 can be prepared, for example, by linking two scFv (single chain Fv) molecules (Huston, J. S. et al., Proc. Natl. Acad. Sci. U.S.A. (1988) 85, 5879-5883; Plickthun "The Pharmacology of Monoclonal Antibodies" Vol.113, Resenburg and Moore ed., Springer Verlag, New York, pp.269-315, (1994)) with a linker and such. The order of the two sets of VH and the two sets of VL to be linked is not particularly limited and may be any order, including for example, the following arrangements.

[VH] linker [VL] linker [VH] linker [VL]
[VL] linker [VH] linker [VH] linker [VL]
[VH] linker [VL] linker [VL] linker [VH]
[VH] linker [VH] linker [VL] linker [VL]
[VL] linker [VL] linker [VH] linker [VH]
[VL] linker [VH] linker [VL] linker [VH]

In the context of the present invention, a preferred sc(Fv)2 arrangement is [VH] linker [VL] linker [VH] linker [VL].

The amino acid sequence of the heavy chain variable region or the light chain variable region may contain substitutions, deletions, additions, and/or insertions. Furthermore, it may also lack portions of heavy chain variable region and/or light chain variable region, or other polypeptides may be added, as long as the binding complex of heavy chain variable regions and light chain variable regions retains its antigen binding activity. Additionally, the variable region may be chimerized or humanized.

In the present invention, the linkers to be used linking for the variable regions of an antibody comprise arbitrary peptide linkers that can be introduced by genetic engineering, synthetic linker compounds, for example, those disclosed in Protein Engineering, 9(3), 299-305, 1996.

In the present invention, preferred linkers are peptide linkers. The length of the polypeptide linkers is not particularly limited and can be suitably selected according to the purpose by those skilled in the art. Normally, the length is 1-100 amino acids, preferably 3-50 amino acids, more preferably 5-30 amino acids, and even more preferably 12-18 amino acids (for example, 15 amino acids).

For example, amino acid sequences for such peptide linkers include:

Ser
Gly·Ser
Gly · Gly · Ser
Ser · Gly · Gly
Gly · Gly · Gly · Ser
Ser · Gly · Gly · Gly
Gly · Gly · Gly · Gly · Ser
Ser· Gly · Gly · Gly · Gly
Gly · Gly · Gly · Gly · Gly · Ser
Ser · Gly · Gly · Gly · Gly · Gly
Gly · Gly · Gly · Gly · Gly · Gly · Ser
Ser·Gly·Gly·Gly·Gly·Gly
(Gly·Gly·Gly·Gly·Ser)n
(Ser·Gly·Gly·Gly·Gly)n

where n is an integer of 1 or more.

Synthetic linker compounds (chemical crosslinking agents) include, crosslinking agents routinely used to crosslink peptides, for example, *N*-hydroxysuccinimide (NHS), disuccinimidyl suberate (DSS), bis(sulfosuccinimidyl) suberate (BS3), dithiobis(succinimidyl propionate) (DSP), dithiobis(sulfosuccinimidyl propionate) (DTSSP), ethylene glycol bis(succinimidyl succinate) (EGS), ethylene glycol bis(sulfosuccinimidyl succinate) (sulfo-EGS), disuccinimidyl tartarate (DST), disulfosuccinimidyl tartarate (sulfo-DST),
bis[2-(succinimidooxycarbonyloxy)ethyl]sulfone (BSOCOES), and
bis[2-(sulfosuccinimidooxycarbonyloxy)ethyl]sulfone (sulfo-BSOCOES). These crosslinking agents are commercially available.

In general, three linkers are required to link four antibody variable regions together. The linkers to be used may be of the same type or different types.

Examples of a preferred sc(Fv)2 of the present invention include, but are not limited to, any one of (a) to (i) indicated below.

    (a) a sc(Fv)2 comprising heavy chain variable regions that comprise CDR1, 2, and 3 consisting of the amino acid sequences of SEQ ID NOs: 3, 4, and 5.
    (b) a sc(Fv)2 comprising light chain variable regions that comprise CDR1, 2, and 3 consisting of the amino acid sequences of SEQ ID NOs: 6, 7, and 8.
    (c) a sc(Fv)2 comprising heavy chain variable regions and light chain variable regions, both of which comprise CDR1, 2, and 3 consisting of the amino acid sequences of SEQ ID NOs: 3, 4, and 5, and SEQ ID NOs: 6, 7, and 8, respectively.
    (d) a sc(Fv)2 comprising heavy chain variable regions comprising the amino acid sequence of SEQ ID NO: 10.
    (e) a sc(Fv)2 comprising light chain variable regions comprising the amino acid sequence of SEQ ID NO: 12.
    (f) a sc(Fv)2 comprising heavy chain variable regions comprising the amino acid sequence of SEQ ID NO: 10 and light chain variable regions comprising the amino acid sequence of SEQ m NO: 12.
    (g) a sc(Fv)2 comprising the amino acid sequence of SEQ ID NO: 14.
    (h) a sc(Fv)2 comprising the amino acid sequence of SEQ ID NO: 2.
    (i) a sc(Fv)2 comprising an amino acid sequence with one or more substitutions, deletions, additions, and/or insertions in the amino acid sequence of any one of (a) to (h), in which the sc(Fv)2 has an activity equivalent to that of the antibodies of the present invention.

SEQ ID NOs: 9 and 10 correspond to the nucleotide sequence and the amino acid sequence of the 2D7 heavy chain variable region, respectively. In the amino acid sequence of SEQ ID: 10, amino acids 50 to 54 correspond to CDR1 (SEQ ID NO: 3), amino acids 69 to 85 correspond to CDR2 (SEQ ID NO: 4), and amino acids 118 to 123 correspond to CDR3 (SEQ ID NO: 5). SEQ ID NOs: 11 and 12 correspond to the nucleotide sequence and the amino acid sequence of the 2D7 light chain variable region, respectively. In the amino acid sequence of SEQ ID: 12, amino acids 46 to 55 correspond to CDR1 (SEQ ID NO: 6), amino acids 71 to 77 correspond to CDR2 (SEQ ID NO: 7), and amino acids 110 to 118 correspond to CDR3 (SEQ ID NO: 8). The nucleotide sequence of a polynucleotide encoding scFv prepared by linking the above-mentioned heavy chain variable region and light chain variable region with a linker is shown in SEQ ID NO: 13; and the amino acid sequence of this scFv is shown in SEQ ID NO: 14. The nucleotide sequence of a polynucleotide encoding the sc(Fv)2 of the present invention is shown in SEQ ID NO: 1; the amino acid sequence of this sc(Fv)2 is shown in SEQ ID NO: 2.

Furthermore, a sc(Fv)2 comprising the amino acid sequence of SEQ ID NO: 2, or a sc(Fv)2 comprising a CDR (or a variable region) in the amino acid sequence of SEQ ID NO: 2 may be humanized or chimerized to reduce heterologous antigenicity against humans. Such artificially modified antibodies can be prepared by using known methods.

In the present invention, the term "functionally equivalent" means that the antibody of interest has an activity equivalent to the sc(Fv)2 comprising the sequence of SEQ ID NO: 2, or the sc(Fv)2 comprising a CDR (or a variable region) in the amino acid sequence of SEQ ID NO: 2 (for example, HLA-A binding activity and cell death-inducing activity). Methods for preparing polypeptides functionally equivalent to a certain polypeptide are well known to those skilled in the art, and include methods of introducing mutations into polypeptides. For example, one skilled in the art can prepare an antibody functionally equivalent to an antibody of the present invention by introducing appropriate mutations into the antibody using site-directed mutagenesis(Hashimoto-Gotoh, T. et al. (1995) Gene 152, 271-275; Zoller, MJ, and Smith, M.(1983) Methods Enzymol. 100, 468-500; Kramer, W. et al. (1984) Nucleic Acids Res. 12, 9441-9456; Kramer W, and Fritz HJ(1987) Methods. Enzymol. 154, 350-367; Kunkel, TA (1985) Proc Natl. Acad. Sci. USA. 82, 488-492; Kunkel (1988) Methods Enzymol. 85, 2763-2766). Amino acid mutations may also occur naturally. Therefore, the antibodies of the present invention also comprise antibodies functionally equivalent to the antibodies of the present invention, wherein the antibodies comprises amino acid sequences with one or more amino acid mutations to the amino acid sequences of the present invention's antibodies.

The number of amino acids that are mutated is not particularly limited, but is generally 30 amino acids or less, preferably 15 amino acids or less, and more preferably 5 amino acids or less (for example, 3 amino acids or less). Preferably, the mutated amino acids conserve the properties of the amino acid side chain from the amino acids that were mutated. Examples of amino acid side chain properties include: hydrophobic amino acids (A, I, L, M, F, P, W, Y, and V), hydrophilic amino acids (R, D, N, C, E, Q, G, H, K, S, and T), amino acids comprising the following side chains: aliphatic side chains (G, A, V, L, I, and P); hydroxyl-containing side chains (S, T, and Y); sulfur-containing side chains (C and M); carboxylic acid- and amide-containing side chains (D, N, E, and Q); basic side chains (R, K, and H); and aromatic ring-containing side chains (H, F, Y, and W) (amino acids are represented by one-letter codes in parentheses). Polypeptides comprising a modified amino acid sequence, in which one or more amino acid residues is deleted, added, and/or substituted with other amino acids, are known to retain their original biological activities (Mark, D. F. et al., Proc. Natl. Acad. Sci. USA (1984) 81, 5662-5666; Zoller, M. J. & Smith, M. Nucleic Acids Research (1982) 10, 6487-6500; Wang, A. et al., Science 224, 1431-1433; Dalbadie-McFarland, G. et al., Proc. Natl. Acad. Sci. (1982) USA 79, 6409-6413).

The antibodies of the present invention also include, antibodies in which several amino acid residues have been added to an amino acid sequence of an antibody of the present invention. Fusion proteins in which such antibodies are fused together with other peptides or proteins are also included in the present invention. A fusion protein can be prepared by ligating a polynucleotide encoding an antibody of the present invention and a polynucleotide encoding another peptide or polypeptide such that the reading frames match, inserting this into an expression vector, and expressing the fusion construct in a host. Techniques known to those skilled in the art are available for this purpose. The peptides or polypeptides to be fused with an antibody of the present invention include, for example, FLAG , (Hopp, T.P. et al.Biotechnology (1988) 6, 1204-1210), 6x His consisting of six His (histidine) residues, 10x His, Influenza hemagglutinin (HA), human c-myc fragment, VSV-GP fragment, p18HIV fragment, T7-tag, HSV-tag, E-tag, SV40T antigen fragment, lck tag, $\alpha$-tubulin fragment, B-tag, Protein C fragment, and such. Examples of other polypeptides to be fused to the antibodies of the present invention include, GST (glutathione-S-transferase), HA (Influenza hemagglutinin), immunoglobulin constant region, $\beta$-galactosidase, MBP (maltose-binding protein), and such. Commercially available polynucleotides encoding these peptides or polypeptides can be fused with polynucleotides encoding the antibodies of the present invention. The fusion polypeptide can be prepared by expressing the fusion construct.

As described below, the antibodies of the present invention may differ in amino acid sequence, molecular weight, and isoelectric point, and may also be different in the presence or absence of sugar chains and conformation, depending on the cell or host producing the antibody or purification method. However, as long as the obtained antibody is functionally equivalent to an antibody of the present invention, it is included in the present invention. For example, when an antibody of the present invention is expressed in a prokaryotic cell such as *E. coli,* a methionine residue is added to the N terminus of the amino acid sequence of the original antibody. The antibodies of the present invention will also include such antibodies.

The antibodies of the present invention may be conjugated antibodies that are bound to various molecules, including, for example, polyethylene glycol (PEG), radioactive substances, and toxins. Such conjugate antibodies can be obtained by chemically modifying the obtained antibodies. Methods for antibody modification are already established in this field (see for example, US5057313, and US5156840). Accordingly, the term "antibody" as used herein includes such conjugate antibodies.

The present invention also provides polynucleotides encoding the antibodies of the present invention, or polynucleotides that hybridize under stringent conditions to the polynucleotides of the present invention and encode antibodies having an activity equivalent to that of the antibodies of this invention. The polynucleotides of the present invention are polymers comprising multiple nucleic bases or base pairs of deoxyribonucleic acids (DNA) or ribonucleic acids (RNA), and are not particularly limited, as long as they encode the antibodies of the present invention. Polynucleotides of the present invention may also contain non-natural nucleotides. The polynucleotides of the present invention can be used to express antibodies using genetic engineering techniques. Furthermore, they can be used as probes in the screening of antibodies functionally equivalent to the antibodies of the present invention. Specifically, DNAs that hybridize under stringent conditions to the polynucleotides encoding the antibodies of the present invention, and encode antibodies having an activity equivalent to that of the antibodies of the present invention, can be obtained by techniques such as hybridization and gene amplification (for example, PCR), using a polynucleotide of the present invention or a portion thereof as a probe. Such DNAs are included in the polynucleotides of the present invention. Hybridization techniques are well known to those skilled in the art (Sambrook, J. et al., Molecular Cloning 2nd ed., 9.47-9.58, Cold Spring Harbor Lab. press, 1989). Conditions for hybridization may include ,for example, those with low stringency. Examples of conditions of low stringency include post-hybridization washing in 0.1x SSC and 0.1% SDS at 42°C, and preferably in 0.1x SSC and 0.1 % SDS at 50°C. More preferable hybridization conditions include those of high stringency. Highly stringent conditions include, for example, washing in 5x SSC and 0.1% SDS at 65°C. In these conditions, the higher the temperature, the more it can be expected that a polynucleotide with a high homology would be obtained. However, several factors such as temperature and salt concentration can influence hybridization stringency, and those skilled in the art can suitably select these factors to accomplish similar stringencies.

An antibody encoded by a polynucleotide obtained by a hybridization and gene amplification technique, and is functionally equivalent to a antibody of the present invention, generally has high homology to the amino acid sequence of the antibody of this invention. The antibodies of the present invention include antibodies that are functionally equivalent and have high amino acid sequence homology to the antibodies of the present invention. The term "high homology" generally means identity at the amino acid level of at least 50% or higher, preferably 75% or higher, more preferably 85% or higher, still more preferably 95% or higher. Polypeptide homology can be determined by the algorithm described in Wilbur, W. J. and Lipman, D. J. Proc. Natl. Acad. Sci. USA 80, 726-730 (1983).

sc(Fv)2 of the present invention can be prepared by methods well known to those skilled in the art. For example, sc(Fv) 2 can be prepared based on the sequence of an HLA-recognizing antibody using genetic recombination techniques well known to those skilled in the art. More specifically, it can be produced by constructing a polynucleotide encoding sc(Fv) 2 based on the sequence of an HLA-recognizing antibody, introducing this into an expression vector, and then expressing in an appropriate host cell (see, for example, Co, M. S. et al., J. Immunol. (1994) 152, 2968-2976; Better, M. and Horwitz, A. H., Methods Enzymol. (1989) 178, 476-496; Pluckthun, A. and Skerra, A., Methods Enzymol. (1989) 178, 497-515; Lamoyi, E., Methods Enzymol. (1986) 121, 652-663; Rousseaux, J. et al., Methods Enzymol. (1986) 121, 663-669; Bird, R. E. and Walker, B. W., Trends Biotechnol. (1991) 9, 132-137).

For the sequence of the HLA-recognizing antibody, a well-known antibody sequence can be used, or an anti-HLA antibody can be prepared by a method well known to those skilled in the art using HLA as the antigen, and then the sequence of this antibody can be obtained and used. Specifically, for example, this can be performed as follows: HLA protein or a fragment thereof is used as a sensitizing antigen to perform immunizations according to conventional immunization methods, the obtained immunocytes are fused with well-known parent cells according to conventional cell fusion methods, and monoclonal antibody-producing cells (hybridomas) are then screened by ordinary screening methods. Antigens can be prepared by known methods, such as a method using baculoviruses (WO98/46777 and such). Hybridomas can be prepared, for example, according to the method of Milstein *et al.* (Kohler, G. and Milstein, C., Methods Enzymol. (1981) 73:3-46). When the antigen has low immunogenicity, immunization can be performed using the antigen bound to immunogenic macromolecules, such as albumin. Thereafter, cDNAs of the variable region (V region) of the antibody are synthesized from the mRNAs of the hybridomas using reverse transcriptase, and the sequences of the obtained cDNAs can be determined by known methods.

Antibodies that recognize HLA are not particularly limited, so long as they bind to HLA; mouse antibodies, rat antibodies, rabbit antibodies, sheep antibodies, human antibodies, and such may be used as necessary. Alternatively, artificially modified, genetically recombinant antibodies, such as chimeric and humanized antibodies, may be used to reduce heterologous antigenicity against humans. These modified antibodies can be produced using known methods.

A chimeric antibody is an antibody comprising the variable regions of the heavy and light chains of an antibody from a non-human mammal such as a mouse, and the constant regions of the heavy and light chains of a human antibody. The chimeric antibody can be produced by linking a polynucleotide encoding the variable regions of the mouse antibody with a polynucleotide encoding the constant regions of the human antibody, incorporating this into an expression vector, and then introducing the vector into a host.

Humanized antibodies are also referred to as "reshaped human antibodies". Such humanized antibodies are obtained by grafting the CDR of an antibody derived from a non-human mammal, for example a mouse, to the CDR of a human antibody, and general gene recombination procedures for this are also known (See, European Patent Application No. 125023 and WO 96/02576). Specifically, a polynucleotide sequence designed to link a murine antibody CDR to the framework region (FR) of a human antibody can be synthesized by PCR, using primers prepared from several oligonucleotides containing overlapping portions of terminal regions (See, methods described in WO98/13388). The obtained polynucleotide is linked to a polynucleotide encoding human antibody constant regions, and this is then integrated into an expression vector, and the antibody is produced by introducing this vector into host cells (see European Patent Application EP 239400, and International Patent Application WO 96/02576). The human antibody FR to be linked via the CDR is selected so the CDR forms a favorable antigen-binding site. To form a suitable antigen-binding site, amino acids in the framework region of the antibody variable region may be substituted in the CDR of the reshaped human antibody, as necessary (Sato, K. et al., 1993, Cancer Res. 53, 851-856). These chimeric antibodies and humanized antibodies can be chimerized, humanized, and such before or after the sc(Fv)2 formation.

Methods for obtaining human antibodies are also known. For example, human lymphocytes can be sensitized *in vitro* with a desired antigen, or with cells expressing the desired antigen, and the sensitized lymphocytes can be fused with human myeloma cells such as U266 to obtain the desired human antibody with antigen-binding activity (Examined Published Japanese Patent Application No. (JP-B) Hei 1-59878). Further, a desired human antibody can be obtained by using a desired antigen to immunize transgenic animals that have a full repertoire of human antibody genes (see International Patent Application WO 93/12227, WO 92/03918, WO 94/02602, WO 94/25585, WO 96/34096, and WO 96/33735). Furthermore, techniques for obtaining human antibodies by panning using a human antibody library are also known. For example, variable regions of human antibodies can be expressed as single chain antibodies (scFvs) on the surface of phages using phage display methods, and phages that bind to antigens can be selected. The polynucleotide sequences that encode the variable regions of the human antibodies binding the antigens can be determined by analyzing the genes of the selected phages. By determining the polynucleotide sequences of the scFvs that bind to the antigens, appropriate expression vectors carrying the sequences can be produced to yield human antibodies. These methods are already known, and the following publications can be referred to: WO 92/01047, WO 92/20791, WO 93/06213, WO 93/11236, WO 93/19172, WO 95/01438, and WO 95/15388.

The antibodies of this invention can be produced by methods well known to those skilled in the art. More specifically, a DNA of an antibody of interest is incorporated into an expression vector. In so doing, the DNA is incorporated into the expression vector and expressed under the control of an expression regulatory region such as an enhancer or promoter. Next, antibodies can be expressed by transforming host cells with this expression vector. In this regard, appropriate combinations of hosts and expression vectors can be used.

The vectors include, for example, M13 vectors, pUC vectors, pBR322, pBluescript, and pCR-Script. In addition to the above vectors, for example, pGEM-T, pDIRECT, and pT7 can also be used for the subcloning and excision of cDNAs. When using vectors to produce the antibodies of this invention, expression vectors are particularly useful. When an expression vector is expressed in *E. coli,* for example, it should have the above characteristics in order to be amplified in *E. coli.* Additionally, when *E. coli* such as JM109, DH5 α, HB101, or XL1-Blue are used as the host cell, the vector preferably has a promoter, for example, a lacZ promoter (Ward et al. (1989) Nature 341:544-546; (1992) FASEB J. 6: 2422-2427), araB promoter (Better et al. (1988) Science 240:1041-1043), or T7 promoter, to allow efficient expression of the desired gene in *E. coli.* Other examples of the vectors include pGEX-5X-1 (Pharmacia), "QIAexpress system" (QIAGEN), pEGFP, and pET (where BL21, a strain expressing T7 RNA polymerase, is preferably used as the host).

Furthermore, the vector may comprise a signal sequence for polypeptide secretion. When producing proteins into the periplasm of *E. coli,* the pelB signal sequence (Lei, S. P. et al. J. Bacteriol. 169:4379 (1987)) may be used as a signal sequence for protein secretion. For example, calcium chloride methods or electroporation methods may be used to introduce the vector into a host cell.

In addition to *E. coli,* expression vectors derived from mammals (e.g., pCDNA3 (Invitrogen), pEGF-BOS (Nucleic Acids Res. (1990) 18(17):5322), pEF, pCDM8), insect cells (e.g., "Bac-to-BAC baculovirus expression system" (GIBCO-BRL), pBacPAK8), plants (e.g., pMH1, pMH2), animal viruses (e.g., pHSV, pMV, pAdexLcw), retroviruses (e.g., pZIPneo), yeasts (e.g., "Pichia Expression Kit" (Invitrogen), pNV11, SP-Q01), and *Bacillus subtilis* (e.g., pPL608, pKTH50) may also be used as vectors for producing the polypeptides of the present invention.

In order to express proteins in animal cells, such as CHO, COS, and NIH3T3 cells, the vector preferably has a promoter necessary for expression in such cells, for example, an SV40 promoter (Mulligan et al. (1979) Nature 277: 108), MMLV-LTR promoter, EF1αpromoter (Mizushima et al. (1990) Nucleic Acids Res. 18:5322), CMV promoter, etc.). It is even more preferable that the vector also carries a marker gene for selecting transformants (for example, a drug-resistance gene enabling selection by a drug such as neomycin and G418). Examples of vectors with such characteristics include pMAM, pDR2, pBK-RSV, pBK-CMV, pOPRSV, pOP 13, and such.

In addition, to stably express a gene and amplify the gene copy number in cells, CHO cells having a defective nucleic acid synthesis pathway can be introduced with a vector containing a DHFR gene (for example, pCHOI) to compensate for the defect, and the copy number may be amplified using methotrexate (MTX). Alternatively, a COS cell, which carries an SV40 T antigen-expressing gene on its chromosome, can be transformed with a vector containing the SV40 replication origin (for example, pcD) for transient gene expression. The replication origin may be derived from polyoma viruses, adenoviruses, bovine papilloma viruses (BPV), and such. Furthermore, to increase the gene copy number in host cells, the expression vector may contain, as a selection marker, an aminoglycoside transferase (APH) gene, thymidine kinase (TK) gene, *E. coli* xanthine guanine phosphoribosyl transferase (Ecogpt) gene, dihydrofolate reductase (dhfr) gene, and such.

Methods for expressing polynucleotides of this invention in animal bodies include methods of incorporating the polynucleotides of this invention into appropriate vectors and introducing them into living bodies by, for example, a retrovirus method, liposome method, cationic liposome method, or adenovirus method. The vectors that are used include adenovirus vectors (for example, pAdexlcw), and retrovirus vectors (for example, pZIPneo), but are not limited thereto. General genetic manipulations such as inserting the polynucleotides of this invention into vectors can be performed according to conventional methods (Molecular Cloning, 5.61-5.63). Administration to living bodies can be carried out by *ex vivo* or *in vivo* methods.

Furthermore, the present invention provides host cells into which a vector of this invention is introduced. The host cells are not particularly limited; for example, *E. coli* and various animal cells are available for this purpose. The host cells of this invention may be used. for example, as production systems to produce and express the antibodies of the present invention. *In vitro and in vivo* production systems are available for polypeptide production systems. Production systems that use eukaryotic cells or prokaryotic cells are examples *of in* vitro production systems.

Eukaryotic cells that can be used include, for example, animal cells, plant cells, and fungal cells. Known animal cells include: mammalian cells, for example, CHO (J. Exp. Med. (1995)108, 945), COS, 3T3, myeloma, BHK (baby hamster kidney), HeLa, Vero, amphibian cells such as *Xenopus laevis* oocytes (Valle, et al. (1981) Nature 291, 358-340), or insect cells (e.g., Sf9, Sf21, and Tn5). CHO cells in which the DHFR gene has been deleted, such as dhfr-CHO (Proc.

Natl. Acad. Sci. USA (1980) 77, 4216-4220) and CHO K-1 (Proc. Natl. Acad. Sci. USA (1968) 60, 1275), are particularly preferable for use as CHO cells. Of the animal cells, CHO cells are particularly favorable for large-scale expression. Vectors can be introduced into a host cell by, for example, calcium phosphate method, DEAE-dextran method, method using cationic liposome DOTAP (Boehringer-Mannheim), electroporation methods, lipofection methods, etc.

Plant cells include, for example, Nicotiana tabacum-derived cells known as polypeptide production systems. Calluses may be cultured from these cells. Known fungal cells include yeast cells, for example, the genus Saccharomyces, such as *Saccharomyces cerevisiae;* and filamentous fungi, for example, the genus Aspergillus such as *Aspergillus niger.*

Bacterial cells can be used in prokaryotic production systems. Examples of bacterial cells include *E. coli* (for example, JM109, DH5α, HB101 and such); and *Bacillus subtilis.*

Antibodies can be obtained by transforming the cells with a polynucleotide of interest, then culturing these transformants *in vitro.* Transformants can be cultured using known methods. For example, DMEM, MEM, RPMI 1640, or IMDM may be used as the culture medium for animal cells, and may be used with or without serum supplements such as fetal calf serum (FCS). Serum-free cultures are also acceptable. The preferred pH is about 6 to 8 over the course of culturing. Incubation is typically carried out at a temperature of about 30 to 40°C for about 15 to 200 hours. Medium is exchanged, aerated, or agitated, as necessary.

On the other hand, production systems using animal or plant hosts may be used as systems for producing polypeptides *in vivo.* For example, a polynucleotide of interest may be introduced into an animal or plant, and the polypeptide produced in the body of the animal or plant is then recovered. The "hosts" of the present invention include such animals and plants.

When using animals, there are production systems using mammals or insects. Mammals such as goats, pigs, sheep, mice, and cattle may be used (Vicki Glaser SPECTRUM Biotechnology Applications (1993)). Alternatively, the mammals may be transgenic animals.

For example, a polynucleotide of interest may be prepared as a fusion gene with a gene encoding a polypeptide specifically produced in milk, such as the goat β-casein gene. Polynucleotide fragments containing the fusion gene are injected into goat embryos, which are then introduced back to female goats. The desired antibody can then be obtained from milk produced by the transgenic goats, which are born from the goats that received the embryos, or from their offspring. Appropriate hormones may be administered to increase the volume of milk containing the polypeptide produced by the transgenic goats (Ebert, K.M. et al., Bio/Technology 12, 699-702 (1994)).

Insects, such as silkworms, may also be used. Baculoviruses carrying a polynucleotide of interest can be used to infect silkworms, and the antibody of interest can be obtained from their body fluids (Susumu, M. et al., Nature 315, 592-594 (1985)).

When using plants, tobacco can be used, for example. When tobacco is used, a polynucleotide of interest may be inserted into a plant expression vector, for example, pMON 530, and then the vector may be introduced into a bacterium such as *Agrobacterium tumefaciens.* The bacteria are then used to infect tobacco, such as *Nicotiana tabacum,* and the desired polypeptides are recovered from the leaves (Julian K.-C. Ma et al., Eur. J. Immunol. 24, 131-138 (1994)).

The resulting antibodies of this invention may be isolated from the inside or outside (such as the medium) of host cells, and purified as substantially pure and homogenous antibodies. Any standard method for isolating and purifying antibodies may be used, and methods are not limited to any specific method. Antibodies may be isolated and purified by selecting an appropriate combination of, for example, chromatographic columns, filtration, ultrafiltration, salting out, solvent precipitation, solvent extraction, distillation, immunoprecipitation, SDS-polyacrylamide gel electrophoresis, isoelectric focusing, dialysis, recrystallization, and others.

Chromatography includes, for example, affinity chromatography, ion exchange chromatography, hydrophobic chromatography, gel filtration, reverse-phase chromatography, and adsorption chromatography (Strategies for Protein Purification and Characterization: A Laboratory Course Manual. Ed Daniel R. Marshak et al., Cold Spring Harbor Laboratory Press, 1996). These chromatographies can be carried out using liquid phase chromatographies such as HPLC and FPLC. Examples of columns used for affinity chromatography include protein A columns and protein G columns. Columns using protein A column include, for example, Hyper D, POROS, Sepharose F. F. (Pharmacia). The present invention also includes antibodies that are highly purified using these purification methods.

In the present invention, the antigen-binding activity of the prepared antibodies (Antibodies A Laboratory Manual. Ed Harlow, David Lane, Cold Spring Harbor Laboratory, 1988) can be measured using well known techniques. For example, ELISA (enzyme linked immunosorbent assay), EIA (enzyme immunoassay), RIA (radioimmunoassay), or fluoroimmunoassay may be used.

The present inventors discovered that the antibodies of the present invention induce cell death. Based on this finding, the present invention provides, cell death-inducing agents or cell growth inhibitors comprising an antibody of the present invention as an active ingredient. The present inventors previously discovered that diabodies prepared by reducing molecular weight of an anti-HLA antibody have an anti-tumor effect against a human myeloma model animal (WO2004/033499). Furthermore, the cell death-inducing activity of the antibodies of the present invention is considered to have a significant effect, particularly in activated T cells or B cells. Accordingly, similar to diabodies, antibodies of the present invention would be particularly effective for treating or preventing tumors such as cancers (specifically blood tumors) and autoimmune diseases. The present invention also provides anti-tumor agents and therapeutic agents for autoimmune diseases, which comprise an antibody of the present invention as an active ingredient.

The antibodies of the present invention can be directly administered to patients, or administered as pharmaceutical compositions formulated by known pharmaceutical methods. For example, they may be administered orally, as tablets, capsules, elixirs, or microcapsules, sugar-coated as necessary; or parenterally, in the form of injections of sterile solutions or suspensions prepared with water or other pharmaceutically acceptable liquids. For example, they may be formulated by appropriately combining them with pharmaceutically acceptable carriers or media, more specifically, sterilized water or physiological saline solutions, vegetable oils, emulsifiers, suspending agents, surfactants, stabilizers, flavoring agents, excipients, vehicles, preservatives, binding agents, and such, and mixing them at a unit dosage form required for generally accepted pharmaceutical practice. The amount of active ingredient in the formulation is such that appropriate doses within indicated ranges are achieved.

Additives that can be mixed into tablets and capsules include, for example, binding agents such as gelatin, cornstarch, tragacanth gum, and gum arabic; excipients such as crystalline cellulose; swelling agents such as cornstarch, gelatin, alginic acid; lubricants such as magnesium stearate; sweeteners such as sucrose, lactose, or saccharine; and flavoring agents such as peppermint and *Gaultheria adenothrix* oils, or cherry. When the unit dosage form is a capsule, liquid carriers, such as oils and fats, can be further included in the above-indicated materials. Sterile compositions to be injected can be formulated using a vehicle such as distilled water used for injection, according to standard protocols.

Aqueous solutions used for injections include, for example, physiological saline and isotonic solutions comprising glucose or other adjunctive agents such as D-sorbitol, D-mannose, D-mannitol, and sodium chloride. They may also be combined with appropriate sntnbilizing agents, such as alcohol, and specifically, ethanol, polyalcohol such as propylene glycol or polyethylene glycol, or non-ionic detergent such as polysorbate 80™ or HCO-50, as necessary.

Oil solutions include sesame oils and soybean oils, and can be combined with solubilizing agents such as benzyl benzoate or benzyl alcohol. Injection solutions may also be formulated with buffers, for example, phosphate buffers or sodium acetate buffers; analgesics, for example, procaine hydrochloride; stabilizers, for example, benzyl alcohol or phenol; or anti-oxidants. The prepared injections are typically aliquoted into appropriate ampules.

Administration to patients may be performed, for example by intra-arterial injection, intravenous injection, or subcutaneous injection, alternatively by intranasal, transbronchial, intramuscular, transdermal, or oral administration using methods well known to those skilled in the art. Doses vary depending on the body weight and age of the patient, method of administration and such; nevertheless, those skilled in the art can appropriately select suitable doses. Furthermore, if a compound can be encoded by a polynucleotide, the polynucleotide may be incorporated into a gene therapy vector to carry out gene therapy. Doses and administration methods vary depending on the body weight, age, and symptoms of patients, but, again, they can be appropriately selected by those skilled in the art.

A single dose of an antibody of this invention varies depending on the target of administration, the target organ, symptoms, and administration method. However, an ordinary adult dose (with a body weight of 60 kg) in the form of an injection is approximately 0.1 to 1000 mg, preferably approximately 1.0 to 50 mg, and more preferably approximately 1.0 to 20 mg per day, for example.

When administered parenterally, a single dose varies depending on the target of administration, the target organ, symptoms, and administration method; however in the form of an injection, for example, a single dose of approximately 0.01 to 30 mg, preferably approximately 0.1 to 20 mg, and more preferably approximately 0.1 to 10 mg per day may be advantageously administered intravenously to an ordinary adult (with a body weight of 60 kg). For other animals, a converted amount based on the amount for a body weight of 60 kg, or a converted amount based on the amount for a body surface area can be administered.

Furthermore, the present invention relates to methods for inducing cell death by using antibodies of the present invention. More specifically, it relates to methods for inducing cell death by contacting the cells with antibodies of the present invention.

All prior-art documents cited herein are incorporated by reference in their entirety.

Examples

The present invention is illustrated in more detail below with reference to the following examples.

[Example 1] Production of expression vectors for 2D7 sc(Fv)2-type diabodies

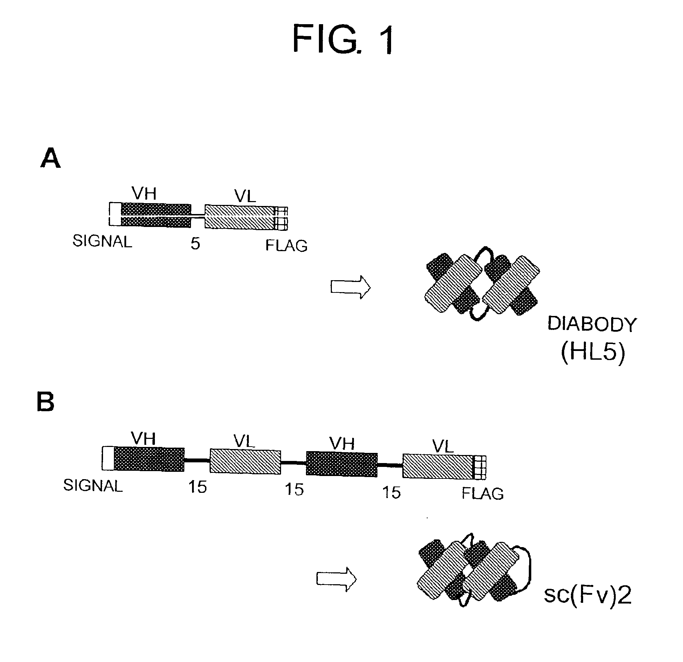

As already described in WO2004/033499, it has been demonstrated that modification of a 2D7 monoclonal antibody against HLA class I to a low molecular weight antibody (2D7 diabody) in which the heavy chain and light chain variable regions are linked with a 5-mer linker (Fig. 1A), dramatically increases cell death-inducing activity against myeloma cells. This 2D7 diabody was further modified to a sc(Fv)2 form, which is thought to be structurally more stable (Fig. 1B), and then the cell death inducing activity was compared with that of the conventional diabody (HL5).

To arrange the heavy chain variable region sequence (VH) and the light chain variable region sequence (VL) of the 2D7 antibody in the order of VH-VL-VH-VL , a DNA expression vector encoding 2D7sc(Fv)2 in which these sequences are linked by a 15-mer linker (GlyGlyGlyGlySerGlyGlyGlyGlySerGlyGlyGlyGlySer) was produced by the following procedure.

2D7 diabody (HL5) expression vector produced according to the method of WO2004/033499 by linking VH-VL with a 5-mer linker (GlyGlyGlyGlySer), was used as a template for a PCR reaction using primer 2D7DBH1 (SEQ ID NO: 15) and primer 2D7PA2 (SEQ ID NO: 16) to amplify fragment A. Similarly, a PCR reaction was performed using primer 2D7PA3 (SEQ ID NO: 17) and primer 2D7PA5 (SEQ ID NO: 18) to amplify fragment B. The obtained fragments A and B were mixed in the same tube, and the two fragments were linked by conducting a PCR-recombination reaction. This yielded the DNA fragment "2D7 diabody HL15-1" comprising a VH signal sequence at the N terminal, in which VH-VL is linked by a 15-mer linker.

Subsequently, using 2D7 diabody (HL5) expression vector as the template, a PCR reaction was performed using primer 2D7PA6 (SEQ ID NO: 19) and primer 2D7PA2 (SEQ ID NO: 16) to amplify fragment C. Similarly, a PCR reaction was performed using primer 2D7PA3 (SEQ ID NO: 17) and primer 2D7DBL2 (SEQ ID NO: 20) to amplify fragment D. The obtained fragments C and D were mixed in the same tube, and the two fragments were linked by conducting a PCR-recombination reaction. This yielded the DNA fragment "2D7diabody HL15-2" comprising a Flag-tag region at the C terminal, in which VH-VL is linked by a 15-mer linker.

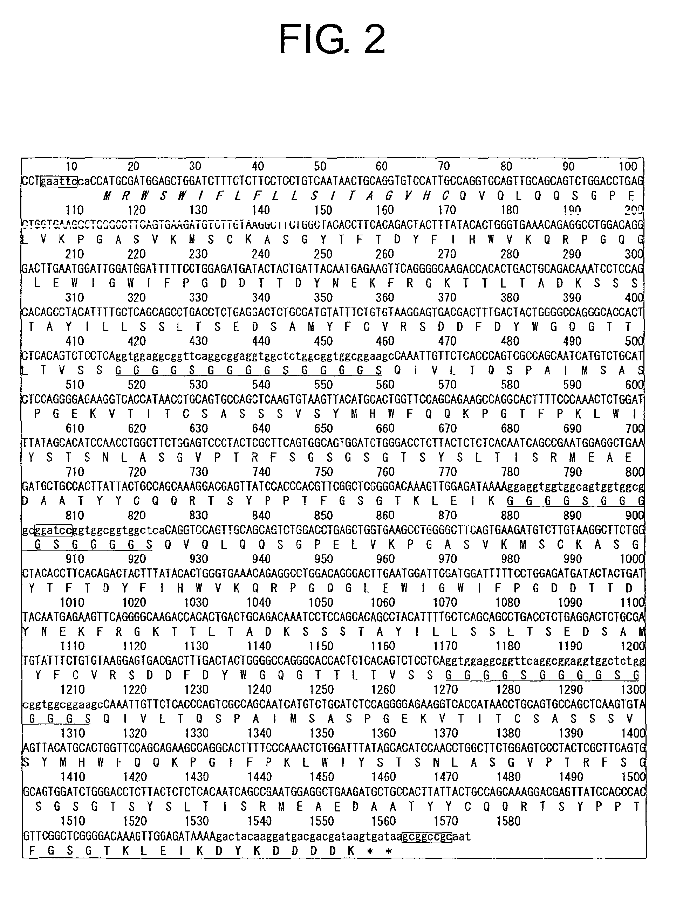

The two DNA fragments obtained by the above-mentioned reactions, that is, "2D7 diabody HL15-1" DNA fragment and "2D7 diabody HL15-2" DNA fragment were digested using EcoRI-BamHI and BamHI-NotI, respectively, and both DNA fragments were inserted into expression vector pCXND3 that had been digested and cleaved using EcoRI-NotI. The nucleotide sequence of the inserted DNA was analyzed to confirm that the cDNA encoding signal-VH(15)VL (15)VH(15)VL-Flag has been inserted between EcoRI-NotI ofpCXND3 as intended, and the construction of the 2D7sc (Fv)2 expression vector (pCXND3-2D7sc(Fv)2) was completed. The nucleotide sequence (SEQ ID NO: 1) and the amino acid sequence (SEQ ID NO: 2) of 2D7sc(Fv)2 are shown in Fig. 2.

[Example 2] Establishment of 2D7sc(Fv)2-producing expression cell lines

20 $\mu$g of linearized pCXND3-2D7sc(Fv)2 obtained by digesting the plasmid with PvuI was introduced into CHO cells (DG44 cell line) by electroporation as described below.

DG44 cells cultured in CHO-S-SFM-II medium (Invitrogen) were washed twice with ice-cold PBS, and then suspended in PBS to a concentration of 1x $10^7$ cells/mL. 20 $\mu$g of the above-mentioned plasmid was mixed with this suspension, and then treated with an electric pulse (1.5 KV, 25 $\mu$FD). The cells were diluted into appropriate ratios, plated onto a 96-well plate, and cultured in CHO-S-SFM-II medium in the presence of G418 (Invitrogen) at a final concentration of 500 $\mu$g/ml. Approximately 30 clones were selected from the grown single colonies, and the expression levels of 2D7sc(Fv)2 in these culture supernatants were investigated by Western blotting, using anti-FLAG antibody (Sigma). The clone with the highest expression level was cultured in nucleic acid-free CHO-S-SFM II medium (Invitrogen) containing 5 nM MTX to expand culture scale. The resulting cell line was regarded as a highly productive cell line.

[Example 3] Large-scale purification of 2D7sc(Fv)2

A sub-confluent, 2D7sc(Fv)2 highly producing CHO cell line in a T-125 flask was transferred to a roller bottle (250 ml of CHO-S-SFM II medium/bottle) to achieve a concentration of 1x $10^5$ cells/mL. The cells were cultured at 37°C and the culture supernatant was collected after 6 days. Dead cells were removed by centrifugation, and the solution was passed through a 0.45 $\mu$m filter and then used for purification.

Purification of 2D7sc(Fv)2 was carried out as follows.

First, the collected culture supernatant was applied to a hydroxyapatite column (microprep ceramic Hydroxyapatite type I, Bio-Rad) equilibrated with buffer A (20 mM Na-phosphate pH6.8). After washing the column with buffer A, 2D7sc(Fv)2 was eluted using buffer C (250 mM Na-phosphate pH6.8). The fraction containing 2D7sc(Fv)2 was diluted with an equal amount of buffer A, and then this was applied to Anti-Flag M2 agarose affinity column (Bio-Rad). This column was washed with buffer C (50 mM tris-HCl pH7.4, 150 mM NaCl, 0.01 % Tween 20), and then 2D7sc(Fv)2 was eluted with buffer D (100 mM Glycine H3.5, 0.01 % Tween 20). The collected sample was immediately neutralized with Tris-HCl pH8.0 to obtain a final concentration of 25 mM. Thereafter, this fraction was concentrated using centriprep YM-10 (AMICON), and then purified by gel filtration chromatography using Superdex200HR (26/60) column (Amersham Pharmacia).

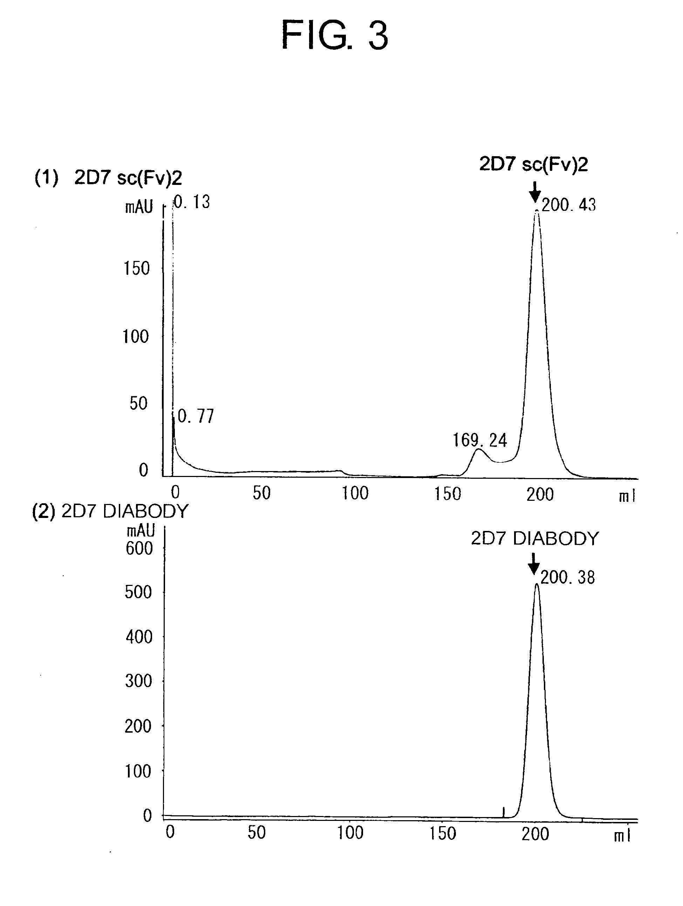

Purification by gel filtration chromatography was performed using PBS containing 0.01% Tween 20. The chromatogram obtained when eluting the sample is shown in Fig. 3. Low-molecular weight antibodies produced from the CHO cell line producing high levels of 2D7sc(Fv)2 mostly have an elution peak at around the molecular weight of 52 Kd (see Fig. 3 (1)), which completely matches the peak of the 2D7 diabody also eluted at 52Kd (see Fig. 3 (2)). Therefore, 2D7sc(Fv)2 constructed in the present invention was deemed to have the structure indicated in Fig. 1B (sc(Fv)2 structure formed by intramolecular folding of a single chain antibody) as initially intended.

Only the 52 Kd peak fraction separated by gel filtration chromatographic purification was collected, and this was used as the 2D7sc(Fv)2 protein sample. SDS electrophoresis and silver staining using a portion of the collected sample was performed to confirm that the desired protein was purified to 100% purity. The collected purified sample was concentrated using Centriprep YM-10 (AMICON) and was used as a purified 2D7sc(Fv)2 sample in the following experiments.

[Example 4] Measurement of cell death-inducing activity of 2D7sc(Fv)2

Human myeloma cell line ARH77 cells were plated onto 24-well plates at $1 \times 10^5$ cells/well in RPMI1640 medium (Invitrogen) containing 10% FCS. Purified 2D7sc(Fv)2 was added to these cells to 100 ng/mL and to 250 ng/mL. As a comparative sample, purified 2D7 diabody (HL5) was added to a separate well under the same conditions. After culturing at 37°C for 3 hours, each type of cells was collected, and the cells were suspended in PI solution (5 $\mu$g/mL PI, 2% FCS/PBS). Following incubation in the dark at room temperature for 15 minutes, the proportion of PI-stained dead cells was measured by flow cytometry (EPICS ELITE, COULTER).

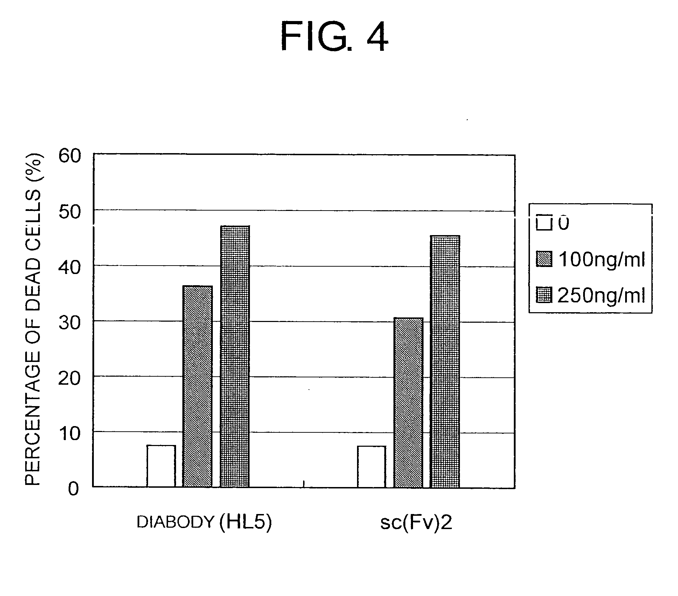

The results showed that 2D7sc(Fv)2 has the activity of inducing cell death in a concentration-dependent manner. It also showed that the level of activity is equivalent to that of the 2D7 diabody (HL5) in which the VH and VL are linked by a 5-mer linker. The above-mentioned results showed that 2D7sc(Fv)2 and 2D7 diabody (HL5) have an equivalent *in vitro* cell death-inducing activity(Fig. 4).

[Example 5] Measurement of cell growth inhibitory activity of 2D7sc(Fv)2

Human EBV-transformed B cell line IM9 cells (ATCC), and human Burkitt lymphoma-derived cell line HS-Sultan cells diluted in RPMI1640 medium containing 10% FCS were plated onto 96-well plates at $3 \times 10^3$ cells/well, and $1 \times 10^4$ cells/well, respectively. Purified 2D7sc(Fv)2 and 2D7 diabody (HL5) were added to these cells to final concentrations of 0, 0.0032, 0.016, 0.08, 0.4, and 2 $\mu$g/mL, and the cells were cultured at 37°C. After culturing for 3 days, the number of viable cells was measured using Cell Counting Kit WST-8 (Dojin Kagaku).

The percentage of viable cells was calculated based on the formula:

$$\text{Percentage of viable cells (\%)} = \text{(Number of viable cells cultured in the presence of an antibody)}$$
$$/ \text{(Number of viable cells cultured in the absence of an antibody),}$$

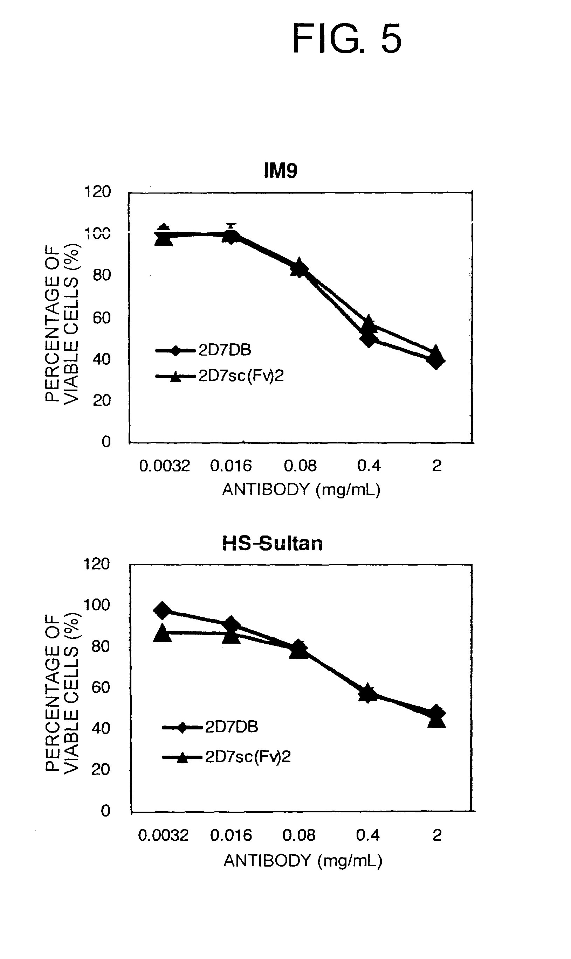

The obtained value was then multiplied by 100 (the vertical axis of Fig. 5).

The results showed that 2D7sc(Fv)2 inhibits the growth of IM9 cells and HS-Sultan cells in a concentration-dependent manner, and has a cell growth inhibitory activity equivalent to that of the 2D7 diabody.

[Example 6] Preparation of radiolabeled 2D7sc(Fv)2 and radiolabeled 2D7 diabody (HL5)

The base of a round-bottom polyethylene tube was cut out such that it had a depth of approximately 1 cm and 20 mmol/L phosphate buffer (pH7.0) containing 250 mmol/L NaCl, 0.05 vol% Tween 20, Na$^{125}$I solution, and a 2D7sc (Fv)2 solution or a 2D7 diabody (HL5) solution were added to this base. A filter paper (5x 5 mm) that had been soaked in 0.15 mol/L NaCl solution and then dried was placed on a cover glass, and the filter paper was impregnated with 32 mg/mL Chloramine T solution, and used to seal the reaction tube such that the filter paper was on the cover glass facing the interior of the tube.

After letting the tube stand for 5 minutes at room temperature, the filter paper was replaced with a filter paper newly impregnated with 32 mg/mL Chloramine T, which was similarly placed to seal the reaction tube. This was then left to stand for another 5 minutes at room temperature.

After adding PBS(-) containing 0.05 vol% Tween 20 to the reaction solution, the reaction solution was loaded onto a PD-10 column (Amersham Pharmacia) equilibrated with PBS(-) containing 0.05 vol% Tween 20, and then eluted with PBS(-) containing 0.05 vol% Tween 20 to remove the unincorporated $^{125}$I. Radioactive iodine-labeled 2D7sc(Fv)2 and radioactive iodine-labeled 2D7 diabody (HL5) were prepared by a further purification by gel filtration through a Superdex200 10/300GL column (Amersham Pharmacia).

[Example 7] Changes in plasma radioactivity concentration due to single intravenous administration of radiolabeled 2D7sc(Fv)2 and radiolabeled 2D7 diabody (HL5) to mice

Male mice (C. B-17/Icr Scid Jcl, Clea Japan) were given single tail intravenous injections of 1 mg/5 MBq/kg of radiolabeled 2D7sc(Fv)2 and radiolabeled 2D7 diabody (HL5). Then, 15 minutes, 30 minutes, and 1, 2, 4, 8, and 24 hours after administration, the mice were subjected to celiotomy incision under etherization, and blood was collected from the heart using a heparin treated Terumo syringe with a 25G needle. The collected blood was immediately centrifuged at 12,000 rpm at 4°C for 5 minutes, and the plasma was separated.

The radioactivity of the plasma samples was measured using a $\gamma$-counter. The radioactivity of the solution that was administered was measured at the same time, and the specific radioactivity of the radiolabeled sample in the administered solution was determined. Based on this, the total plasma radioactivity concentration was then calculated After measuring radioactivity, purified water and 25 w/v% TCA solution was added to the plasma samples, and the solution was stirred and centrifuged at 3000 rpm for 10 minutes at 4°C. The supernatant was removed by suction using an aspirator, and the radioactivity of the precipitate was measured. The percentage of radioactivity of the precipitate with respect to the total radioactivity was multiplied to the total plasma radioactivity to calculate the radioactivity concentration of plasma TCA-precipitable fractions.

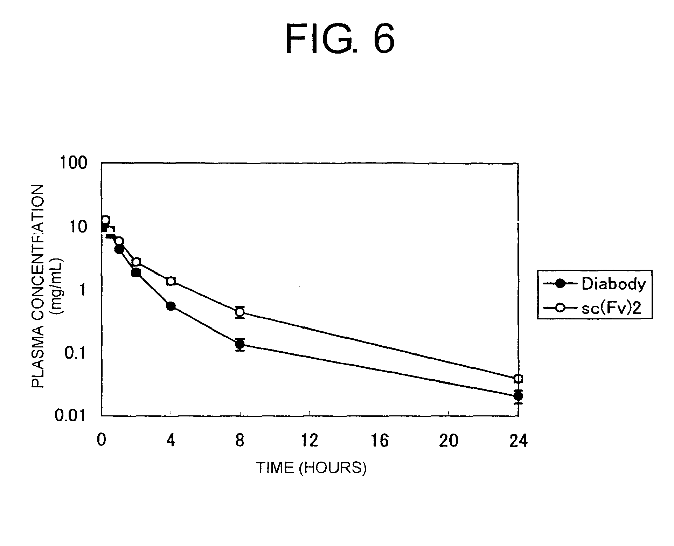

The radioactivity concentration of the plasma TCA-precipitable fractions decreased in a biphasic manner in both radiolabeled 2D7sc(Fv)2 and radiolabeled 2D7 diabody (HL5)-administered groups (Fig. 6). Radiolabeled 2D7sc (Fv)2 exhibited higher radioactivity concentration of plasma TCA-precipitable fractions than radiolabeled 2D7diabody (HL5), and the half life of the elimination phase was 2.30 hours and 1.64 hours, respectively. Thus, 2D7sc(Fv)2 exhibited a longer half life than 2D7 diabody (HL5).

Industrial Applicability

Structural modification of a HLA class IA-recognizing low-molecular weight antibody (diabody) into sc(Fv)2 improves stability of the antibody in blood, while maintaining a high cell death-inducing activity and cell growth inhibitory activity, enabling exertion of excellent drug efficacy *in vivo.*

**Claims**

1. An antibody comprising two heavy chain variable regions and two light chain variable regions, wherein the antibody is a single chain polypeptide having a binding activity against human leukocyte antigen (HLA).

2. The antibody of claim 1, wherein the two heavy chain variable regions and two light chain variable regions are arranged in the order of heavy chain variable region, light chain variable region, heavy chain variable region, and light chain variable region, starting from the N terminus of the single chain polypeptide.

3. The antibody of claim 1 or 2, wherein the two heavy chain variable regions and two light chain variable regions are linked by a linker.

4. The antibody of claim 3, wherein the linker comprises 15 amino acids.

5. The antibody of any one of claims 1 to 4, wherein HLA is HLA class I.

6. The antibody of claim 5, wherein HLA class I is HLA-A.

7. The antibody of any one of claims 1 to 6, wherein the antibody is sc(Fv)2.

8. An sc(Fv)2 comprising heavy chain variable regions that comprise CDR1, 2, and 3 consisting of the amino acid sequences of SEQ ID NOs: 3, 4, and 5.

9. An sc(Fv)2 comprising light chain variable regions that comprise CDR 1, 2, and 3 consisting of the amino acid sequences of SEQ ID NOs: 6, 7, and 8.

10. An sc(Fv)2 comprising heavy chain variable regions that comprise CDR1, 2, and 3 consisting of the amino acid sequences of SEQ ID NOs: 3, 4, and 5, and light chain variable regions that comprise CDR 1, 2, and 3 consisting of the amino acid sequences of SEQ ID NOs: 6, 7, and 8.

**11.** An sc(Fv)2 comprising heavy chain variable regions that comprise the amino acid sequence of SEQ ID NO: 10.

**12.** An sc(Fv)2 comprising light chain variable regions that comprise the amino acid sequence of SEQ ID NO: 12.

**13.** An sc(Fv)2 comprising heavy chain variable regions that comprise the amino acid sequence of SEQ ID NO: 10, and light chain variable regions that comprise the amino acid sequence of SEQ ID NO: 12.

**14.** An sc(Fv)2 comprising the amino acid sequence of SEQ ID NO: 14.

**15.** An sc(Fv)2 comprising the amino acid sequence of SEQ ID NO: 2.

**16.** An sc(Fv)2 comprising an amino acid sequence with one or more amino acid substitutions, deletions, additions, and/or insertions in the amino acid sequence of any one of claims 8 to 15, wherein the sc(Fv)2 also has an activity equivalent to that of the antibody of any one of claims 8 to 15.

**17.** A polynucleotide encoding the antibody of any one of claims 1 to 16.

**18.** A polynucleotide that hybridizes with the polynucleotide of claim 17 under stringent conditions, and encodes an antibody having an activity equivalent to the antibody of any one of claims 1 to 16.

**19.** A vector comprising the polynucleotide of claim 17 or 18.

**20.** A host cell carrying the polynucleotide of claim 17 or 18, or the vector of claim 19.

**21.** A method for producing the antibody of any one of claims 1 to 16, wherein the method comprises the steps of:

(a) preparing an HLA-recognizing antibody;
(b) producing a polynucleotide encoding the antibody of any one of claims 1 to 16 based on the sequence of the antibody prepared in (a);
(c) constructing a vector comprising the polynucleotide of (b);
(d) introducing the vector of (c) into host cells; and
(e) culturing the host cells of (d).

**22.** A cell death-inducing agent comprising the antibody of any one of claims 1 to 16 as an active ingredient.

**23.** The cell death-inducing agent of claim 22, wherein the agent has cell death inducing activity against B cells or T cells.

**24.** The cell death-inducing agent of claim 23, wherein the B cells or T cells are activated B cells or activated T cells.

**25.** A cell growth inhibitory agent comprising the antibody of any one of claims 1 to 16 as an active ingredient.

**26.** An antitumor agent comprising the antibody of any one of claims 1 to 16 as an active ingredient.

**27.** The antitumor agent of claim 26, wherein the tumor is a blood tumor.

**28.** A therapeutic agent for autoimmune diseases, wherein the agent comprises the antibody of any one of claims 1 to 16 as an active ingredient.

# FIG. 1

**A**

SIGNAL  VH  5  VL  FLAG → DIABODY (HL5)

**B**

SIGNAL  VH  15  VL  15  VH  15  VL  FLAG → sc(Fv)2

# FIG. 2

```
          10        20        30        40        50        60        70        80        90       100
CCTgaattccaCCATGCGATGGAGCTGGATCTTTCTCTTCCTCCTGTCAATAACTGCAGGTGTCCATTGCCAGGTCCAGTTGCAGCAGTCTGGACCTGAG
            M R W S W I F L F L L S I T A G V H C Q V Q L Q Q S G P E
         110       120       130       140       150       160       170       180       190       200
CTGGTGAAGCCTGGGGCTTCAGTGAAGATGTCTTGTAAGGCTTCTGGCTACACCTTCACAGACTACTTTATACACTGGGTGAAACAGAGGCCTGGACAGG
  L V K P G A S V K M S C K A S G Y T F T D Y F I H W V K Q R P G Q G
         210       220       230       240       250       260       270       280       290       300
GACTTGAATGGATTGGATGGATTTTTCCTGGAGATGATACTACTGATTACAATGAGAAGTTCAGGGGCAAGACCACACTGACTGCAGACAAATCCTCCAG
  L E W I G W I F P G D D T T D Y N E K F R G K T T L T A D K S S S
         310       320       330       340       350       360       370       380       390       400
CACAGCCTACATTTTGCTCAGCAGCCTGACCTCTGAGGACTCTGCGATGTATTTCTGTGTAAGGAGTGACGACTTTGACTACTGGGGCCAGGGCACCACT
  T A Y I L L S S L T S E D S A M Y F C V R S D D F D Y W G Q G T T
         410       420       430       440       450       460       470       480       490       500
CTCACAGTCTCCTCAggtggaggcggttcaggcggaggtggctctggcggtggcggaagcCAAATTGTTCTCACCCAGTCGCCAGCAATCATGTCTGCAT
  L T V S S G G G G S G G G G S G G G G S Q I V L T Q S P A I M S A S
         510       520       530       540       550       560       570       580       590       600
CTCCAGGGGAGAAGGTCACCATAACCTGCAGTGCCAGCTCAAGTGTAAGTTACATGCACTGGTTCCAGCAGAAGCCAGGCACTTTTCCCAAACTCTGGAT
  P G E K V T I T C S A S S S V S Y M H W F Q Q K P G T F P K L W I
         610       620       630       640       650       660       670       680       690       700
TTATAGCACATCCAACCTGGCTTCTGGAGTCCCTACTCGCTTCAGTGGCAGTGGATCTGGGACCTCTTACTCTCTCACAATCAGCCGAATGGAGGCTGAA
  Y S T S N L A S G V P T R F S G S G S G T S Y S L T I S R M E A E
         710       720       730       740       750       760       770       780       790       800
GATGCTGCCACTTATTACTGCCAGCAAAGGACGAGTTATCCACCCACGTTCGGCTCGGGGACAAAGTTGGAGATAAAAggaggtggtggcagtggtggcg
  D A A T Y Y C Q Q R T S Y P P T F G S G T K L E I K G G G G S G G G
         810       820       830       840       850       860       870       880       890       900
gcggatccggtggcggtggctcaCAGGTCCAGTTGCAGCAGTCTGGACCTGAGCTGGTGAAGCCTGGGGCTTCAGTGAAGATGTCTTGTAAGGCTTCTGG
  G S G G G G S Q V Q L Q Q S G P E L V K P G A S V K M S C K A S G
         910       920       930       940       950       960       970       980       990      1000
CTACACCTTCACAGACTACTTTATACACTGGGTGAAACAGAGGCCTGGACAGGGACTTGAATGGATTGGATGGATTTTTCCTGGAGATGATACTACTGAT
  Y T F T D Y F I H W V K Q R P G Q G L E W I G W I F P G D D T T D
        1010      1020      1030      1040      1050      1060      1070      1080      1090      1100
TACAATGAGAAGTTCAGGGGCAAGACCACACTGACTGCAGACAAATCCTCCAGCACAGCCTACATTTTGCTCAGCAGCCTGACCTCTGAGGACTCTGCGA
  Y N E K F R G K T T L T A D K S S S T A Y I L L S S L T S E D S A M
        1110      1120      1130      1140      1150      1160      1170      1180      1190      1200
TGTATTTCTGTGTAAGGAGTGACGACTTTGACTACTGGGGCCAGGGCACCACTCTCACAGTCTCCTCAggtggaggcggttcaggcggaggtggctctgg
   Y F C V R S D D F D Y W G Q G T T L T V S S G G G G S G G G G S G
        1210      1220      1230      1240      1250      1260      1270      1280      1290      1300
cggtggcggaagcCAAATTGTTCTCACCCAGTCGCCAGCAATCATGTCTGCATCTCCAGGGGAGAAGGTCACCATAACCTGCAGTGCCAGCTCAAGTGTA
  G G G S Q I V L T Q S P A I M S A S P G E K V T I T C S A S S S V
        1310      1320      1330      1340      1350      1360      1370      1380      1390      1400
AGTTACATGCACTGGTTCCAGCAGAAGCCAGGCACTTTTCCCAAACTCTGGATTTATAGCACATCCAACCTGGCTTCTGGAGTCCCTACTCGCTTCAGTG
  S Y M H W F Q Q K P G T F P K L W I Y S T S N L A S G V P T R F S G
        1410      1420      1430      1440      1450      1460      1470      1480      1490      1500
GCAGTGGATCTGGGACCTCTTACTCTCTCACAATCAGCCGAATGGAGGCTGAAGATGCTGCCACTTATTACTGCCAGCAAAGGACGAGTTATCCACCCAC
  S G S G T S Y S L T I S R M E A E D A A T Y Y C Q Q R T S Y P P T
        1510      1520      1530      1540      1550      1560      1570      1580
GTTCGGCTCGGGGACAAAGTTGGAGATAAAAgactacaaggatgacgacgataagtgataagcggccgcaat
  F G S G T K L E I K D Y K D D D D K * *
```

# FIG. 3

(1) 2D7 sc(Fv)2

2D7 sc(Fv)2

(2) 2D7 DIABODY

2D7 DIABODY

# FIG. 4

# FIG. 5

# FIG. 6

<table>
<tr><td colspan="2" align="center">**INTERNATIONAL SEARCH REPORT**</td><td>International application No.<br>PCT/JP2004/018501</td></tr>
</table>

| | |
|---|---|
| A. CLASSIFICATION OF SUBJECT MATTER<br>Int.Cl⁷ | |

A.  CLASSIFICATION OF SUBJECT MATTER
   Int.Cl$^7$ C07K16/28, C12N15/11, 1/15, 1/19, 1/21, 5/00, C12P21/02,
            A61K39/395, A61P35/00, 35/02, 37/02, 43/00//C12P21/08

According to International Patent Classification (IPC) or to both national classification and IPC

B.  FIELDS SEARCHED

Minimum documentation searched (classification system followed by classification symbols)
   Int.Cl$^7$ C07K16/28, C12N15/11, 1/15, 1/19, 1/21, 5/00, C12P21/02,
            A61K39/395, A61P35/00, 35/02, 37/02, 43/00//C12P21/08

Documentation searched other than minimum documentation to the extent that such documents are included in the fields searched

Electronic data base consulted during the international search (name of data base and, where practicable, search terms used)
   CAPlus(STN), MEDLINE(STN), BIOSIS(STN), WPIDS(STN), JICST FILE(JOIS),
   SwissProt/PIR/GeneSeq, Genbank/EMBL/DDBJ/GeneSeq

C.  DOCUMENTS CONSIDERED TO BE RELEVANT

| Category* | Citation of document, with indication, where appropriate, of the relevant passages | Relevant to claim No. |
|---|---|---|
| P,X | BRUENKE J. et al., "A recombinant bispecific single-chain Fv antibody against HLA class II and FcγRIII (CD16) triggers effective lysis of lymphoma cells", British Journal of Haematology, April 2004, Vol.125, No.2, pages 167 to 179 | 1-7,17-28 |
| Y | SEKIMOTO E. et al., "A recombinant HLA Class I-Specific Single Fv Diabody Induces Cell Death in Human Lymphoid Malignancies.", Blood, November, 2003, Vol.102, No.11, page 933a, Abstract. #3474 | 1-14,16-28 |

| | | | |
|---|---|---|---|
| ☒ | Further documents are listed in the continuation of Box C. | ☐ | See patent family annex. |

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| | |
|---|---|
| Date of the actual completion of the international search<br>11 March, 2005 (11.03.05) | Date of mailing of the international search report<br>29 March, 2005 (29.03.05) |
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

| International application No. |
|---|
| PCT/JP2004/018501 |

C (Continuation).   DOCUMENTS CONSIDERED TO BE RELEVANT

| Category* | Citation of document, with indication, where appropriate, of the relevant passages | Relevant to claim No. |
|---|---|---|
| Y | KIPRIYANOV SM. et al., "Effect of domain order on the activity of bacterially produced bispecific single-chain Fv antibodies", J.Mol.Biol., 2003, June, Vol.330, No.1, pages 99 to 111 | 1-14,16-28 |
| Y | Laurent Genestier, et al., "Fas-Independent Apoptosis of Activated T Cells Induced by Antibodies to the HLA Class I 1 Domain", Blood, 1997, Vol.90, No.9, pages 3629 to 3639 | 1-7,17-28 |
| Y | Hudson PJ. et al., "High avidity scFv multimers: diabodies and triabodies.", J.Immunol.Methods., 1999, Vol.231, No. 1-2, p.177-89., Review | 1-7,17-28 |
| Y | JP 7-503622 A   (The Dow Chemical Co.), 20 April, 1995 (20.04.95), Full description & WO 94/13806 A1          & EP 628078 A & US 5877291 A1 | 1-7,17-28 |
| Y | CO MS., et al., "A humanized antibody specific for the platelet integrin gpIIb/IIIa." J.Immunol., 1994, Vol.152, No.6, p.2968-76. | 1-14,16-28 |
| P,A | WO 2004/033499 A1  (Chugai Pharmaceutical Co., Ltd.), 22 April, 2004 (22.04.04), (Family: none) | 1-28 |

Form PCT/ISA/210 (continuation of second sheet) (January 2004)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004033499 A
- US 5057313 A
- US 5156840 A
- WO 9846777 A
- EP 125023 A
- WO 9602576 A
- WO 9813388 A
- EP 239400 A
- JP 1059878 B
- WO 9312227 A
- WO 9203918 A
- WO 9402602 A
- WO 9425585 A
- WO 9634096 A
- WO 9633735 A
- WO 9201047 A
- WO 9220791 A
- WO 9306213 A
- WO 9311236 A
- WO 9319172 A
- WO 9501438 A
- WO 9515388 A

**Non-patent literature cited in the description**

- **FAYEN et al.** *Int. Immunol.,* 1998, vol. 10, 1347-1358
- **GENESTIER et al.** *Blood,* 1997, vol. 90, 3629-3639
- **GENESTIER et al.** *Blood,* 1997, vol. 90, 726-735
- **GENESTIER et al.** *J. Biol. Chem.,* 1998, vol. 273, 5060-5066
- **WOODLE et al.** *J. Immunol.,* 1997, vol. 158, 2156-2164
- **MATSUOKA et al.** *J. Exp. Med.,* 1995, vol. 181, 2007-2015
- **GOTO et al.** *Blood,* 1994, vol. 84, 1922
- **HUDSON et al.** *J. Immunol. Methods,* 1999, vol. 231, 177-189
- **HUSTON, J. S. et al.** *Proc. Natl. Acad. Sci. U.S.A.,* 1988, vol. 85, 5879-5883
- **PLICKTHUN.** The Pharmacology of Monoclonal Antibodies. Springer Verlag, 1994, vol. 113, 269-315
- *Protein Engineering,* 1996, vol. 9 (3), 299-305
- **HASHIMOTO-GOTOH, T. et al.** *Gene,* 1995, vol. 152, 271-275
- **ZOLLER, MJ ; SMITH, M.** *Methods Enzymol.,* 1983, vol. 100, 468-500
- **KRAMER, W. et al.** *Nucleic Acids Res.,* 1984, vol. 12, 9441-9456
- **KRAMER W ; FRITZ HJ.** *Methods. Enzymol.,* 1987, vol. 154, 350-367
- **KUNKEL, TA.** *Proc Natl. Acad. Sci. USA.,* 1985, vol. 82, 488-492
- **KUNKEL.** *Methods Enzymol.,* 1988, vol. 85, 2763-2766
- **MARK, D. F. et al.** *Proc. Natl. Acad. Sci. USA,* 1984, vol. 81, 5662-5666
- **ZOLLER, M. J. ; SMITH, M.** *Nucleic Acids Research,* 1982, vol. 10, 6487-6500
- **WANG, A. et al.** *Science,* vol. 224, 1431-1433
- **DALBADIE-MCFARLAND, G. et al.** *Proc. Natl. Acad. Sci.,* 1982, vol. 79, 6409-6413
- **HOPP, T.P. et al.** *Biotechnology,* 1988, vol. 6, 1204-1210
- **SAMBROOK, J. et al.** Molecular Cloning. Cold Spring Harbor Lab. press, 1989, 9.47-9.58
- **WILBUR, W. J. ; LIPMAN, D. J.** *Proc. Natl. Acad. Sci. USA,* 1983, vol. 80, 726-730
- **CO, M. S. et al.** *J. Immunol.,* 1994, vol. 152, 2968-2976
- **BETTER, M. ; HORWITZ, A. H.** *Methods Enzymol.,* 1989, vol. 178, 476-496
- **PLUCKTHUN, A. ; SKERRA, A.** *Methods Enzymol.,* 1989, vol. 178, 497-515
- **LAMOYI, E.** *Methods Enzymol.,* 1986, vol. 121, 652-663
- **ROUSSEAUX, J. et al.** *Methods Enzymol.,* 1986, vol. 121, 663-669
- **BIRD, R. E. ; WALKER, B. W.** *Trends Biotechnol.,* 1991, vol. 9, 132-137
- **KOHLER, G. ; MILSTEIN, C.** *Methods Enzymol.,* 1981, vol. 73, 3-46
- **SATO, K. et al.** *Cancer Res.,* 1993, vol. 53, 851-856
- **WARD et al.** *Nature,* 1989, vol. 341, 544-546
- *FASEB J.,* 1992, vol. 6, 2422-2427
- **BETTER et al.** *Science,* 1988, vol. 240, 1041-1043

- **LEI, S. P. et al.** *J. Bacteriol.,* 1987, vol. 169, 4379
- *Nucleic Acids Res.,* 1990, vol. 18 (17), 5322
- **MULLIGAN et al.** *Nature,* 1979, vol. 277, 108
- **MIZUSHIMA et al.** *Nucleic Acids Res.,* 1990, vol. 18, 5322
- *J. Exp. Med.,* 1995, vol. 108, 945
- **VALLE et al.** *Nature,* 1981, vol. 291, 358-340
- *Proc. Natl. Acad. Sci. USA,* 1980, vol. 77, 4216-4220
- *Proc. Natl. Acad. Sci. USA,* 1968, vol. 60, 1275
- **EBERT, K.M. et al.** *Bio/Technology,* 1994, vol. 12, 699-702
- **SUSUMU, M. et al.** *Nature,* 1985, vol. 315, 592-594
- **JULIAN K.-C. MA et al.** *Eur. J. Immunol.,* 1994, vol. 24, 131-138
- Strategies for Protein Purification and Characterization: A Laboratory Course Manual. Cold Spring Harbor Laboratory Press, 1996
- Antibodies A Laboratory Manual. Cold Spring Harbor Laboratory, 1988